(19) Europäisches Patentamt
European Patent Office
Office européen des brevets

(11) EP 1 595 882 A1

(12) **EUROPEAN PATENT APPLICATION**
published in accordance with Art. 158(3) EPC

(43) Date of publication:
**16.11.2005 Bulletin 2005/46**

(21) Application number: **03815453.0**

(22) Date of filing: **19.11.2003**

(51) Int Cl.$^7$: **C07D 473/40**

(86) International application number:
**PCT/JP2003/014703**

(87) International publication number:
**WO 2004/065386 (05.08.2004 Gazette 2004/32)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **24.01.2003 JP 2003016667**

(71) Applicant: **Sumitomo Chemical Company, Limited**
**Tokyo 104-8260 (JP)**

(72) Inventors:
- **KOTSCHY, A., Dept. Gen. and Inorganic Chemistry H-1117 Budapest (HU)**
- **NAGY, A., Dept Gen. and Inorganic Chemistry H-1117 Budapest (HU)**
- **BIR , A.B., Dept Gen. and Inorganic Chemistry H-1117 Budapest (HU)**

(74) Representative: **Duckett, Anthony Joseph et al**
**J.A. Kemp & Co.,**
**14 South Square,**
**Gray's Inn**
**London WC1R 5JJ (GB)**

(54) **2,6-DIHALOGENO-8-SUBSTITUENT-PURINE COMPOUND AND PROCESS FOR PRODUCING THE SAME**

(57) The present invention relates to a compound represented by the formula (1) :

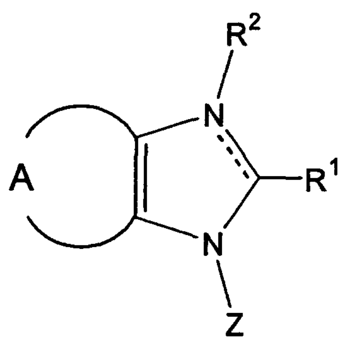

(1)

wherein

-A- is

EP 1 595 882 A1

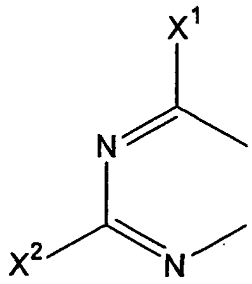

or

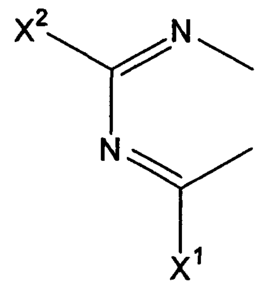

wherein $X^1$ and $X^2$ are each independently a halogen atom,

------ is a single bond or a double bond, and
$R^1$, $R^2$ and Z are each as defined in the description, or a salt thereof, a production method thereof and the like. A 2,6-dihalogeno-8-substituted-purine compound or a salt thereof, which is useful as an intermediate for producing medicaments, can be conveniently produced from a 2,6-dihalogenopurine compound or a salt thereof, and a 2,6-dihalogeno-8-substituted-purine compound or a salt thereof can be easily provided.

## Description

### Technical Field

The present invention relates to a 2,6-dihalogeno-8-substituted-purine compound (including derivatives thereof) or a salt thereof, which is useful as an intermediate for producing medicaments, and a production method thereof.

### Background Art

At present, medicaments containing purine nucleus such as Neuropeptide Y antagonistic inhibitors and the like (e.g., see US patent application publication No. 2002/0058671, US Patent No. 5,576,337 and EP-B-0759441) have been actively developed in the art, and purine compounds that can be synthetic intermediates for such medicaments have also been actively developed.

Of these, a 2,6-dihalogeno-8-substituted-purine compound becomes a common intermediate for various pharmaceutical products since it has halogen atoms at the 2-position and 6-position of the purine nucleus, respectively, and each halogen atom can be substituted with the other substituent. Particularly, it can be extremely useful as an intermediate for producing the above-mentioned medicaments.

As a production method of a 2,6-dihalogeno-8-substituted-purine compound, for example, a method described in US patent application publication No. 2002/0058671 can be mentioned:

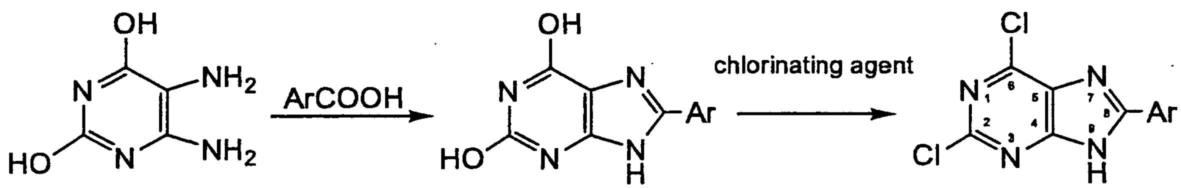


wherein Ar is an aryl optionally having substituent(s) or an heteroaryl optionally having substituent(s), wherein Ar is preferably phenyl, and, for example, the chlorinating agent includes $POCl_3$ and the like.

While the above-mentioned method is based on a concept of introducing a substituent (i.e., Ar substituent in the scheme) upon the purine nucleus construction, it is difficult to introduce a variety of substituents at the 8-position of the purine nucleus easily. In addition, the above-mentioned method comprises the step of introducing chlorine atoms at the 2-position and the 6-position of the purine nucleus using a chlorinating agent such as $POCl_3$ and the like, which makes this method complicated as a production method of a 2,6-dihalogeno-8-substituted-purine compound. Moreover, the above-mentioned method has problems such as a fused ring formation reaction with the pyrimidine derivative and the carboxylic acid requiring extreme reaction conditions.

Moreover, as a method of directly introducing a substituent at the 8-position of the purine nucleus, for example, methods described in Heterocycles, 30 (1), 435 (1990) and J. Heterocyclic Chem., 24, 1551 (1987), and the like can be mentioned.

Heterocycles, 30 (1), 435 (1990) discloses a method of introducing a substituent at the 8-position of the purine nucleus via a reaction of 9-phenyl-9H-purine-2-carbonitrile with Grignard reagent. To be specific, it discloses the following reaction to give a 8-phenyl-purine compound:

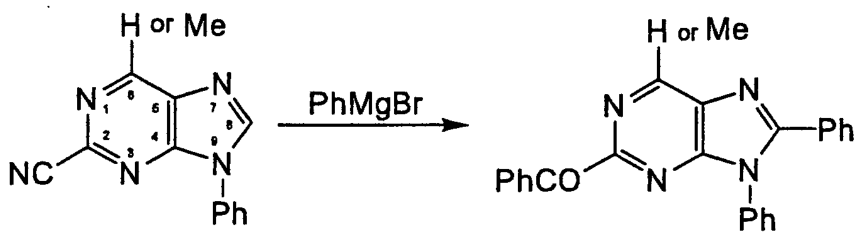


However, the above-mentioned method has problems such as difficult conversion of the phenyl group at the 9-position to the other substituent (e.g., sugar group, etc.), since the phenyl group is attached to the nitrogen atom of the 9-position of the purine nucleus. According to the above-mentioned method, moreover, it allows introduction of the substituent at the 8-position of the purine nucleus, but the utility of the produced purine compound, as an intermediate

for the medicament production, is considerably low, because both the 2-position and the 6-position thereof do not have halogen atoms.

J. Heterocyclic Chem., 24, 1551 (1987) discloses a method of introducing a phenyl group at the 8-position of the purine nucleus via a reaction of 6-halopurine with a phenyl-metal complex. To be specific, it discloses the following three reactions to give a 6-halo-8-phenylpurine compounds:

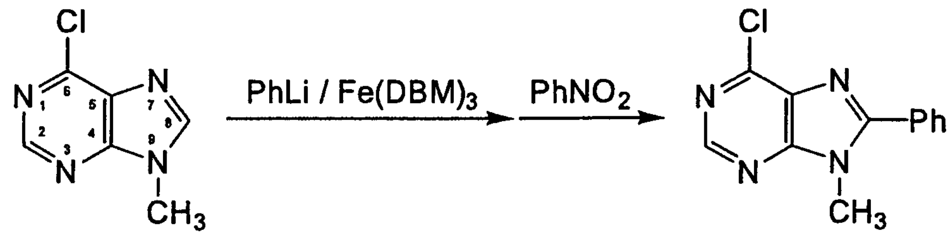


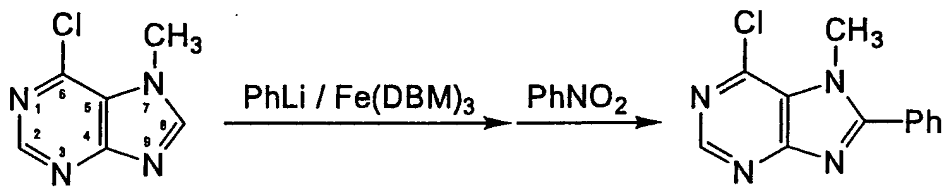


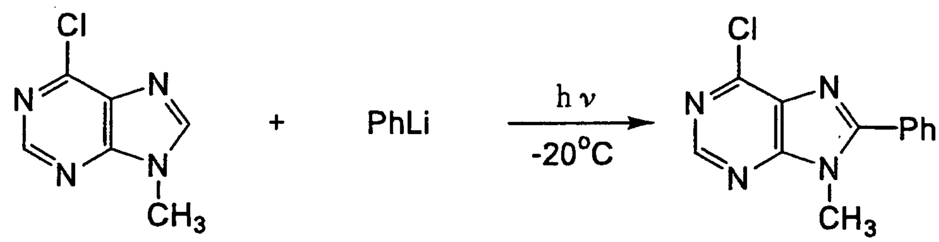


wherein $Fe(DBM)_3$ is tris(dibenzoylmethido) iron (III) (see S. M. Neumann and J. K. Kochi, J. Org. Chem., 40, 599 (1975)), which is a catalyst improving the yield, and $PhNO_2$ (nitrobenzene) is used as an oxidizing reagent.

According to the above-mentioned method, it allows introduction of the substituent at the 8-position of the purine nucleus, but the utility of the produced purine compound, as an intermediate for the medicament production, is low, because the 2-position thereof does not have a halogen atom as well.

Therefore, if a variety of substituents can be easily introduced at the 8-position of a 2,6-dihalogenopurine compound or a salt thereof, a 2,6-dihalogeno-8-substituted-purine compound or a salt thereof, which is useful as an intermediate for producing medicaments, can be conveniently produced, and a desired 2,6-dihalogeno-8-substituted-purine compound or a salt thereof (including derivatives thereof) can be easily provided.

**Disclosure of the Invention**

An object of the present invention is to easily produce a 2,6-dihalogeno-8-substituted-purine compound or a salt thereof, which is useful as an intermediate for the production of medicaments, from a 2,6-dihalogenopurine compound or a salt thereof, and to easily provide a 2,6-dihalogeno-8-substituted-purine compound or a salt thereof.

The present inventors have conducted intensive studies in view of the above-mentioned problems and found a method of conveniently producing various 2,6-dihalogeno-8-substituted-purine compounds, which comprises combining a step of reacting a 2,6-dihalogenopurine compound with an organometallic reagent, and an oxidization step using an oxidizing reagent, and the like, which resulted in the completion of the present invention. Accordingly, the present invention relates to the following [1]-[14].

[1] A compound represented by the formula (1) :

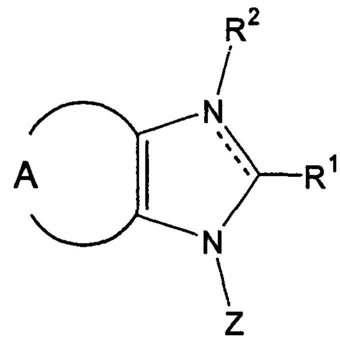 (1)

wherein

-A- is

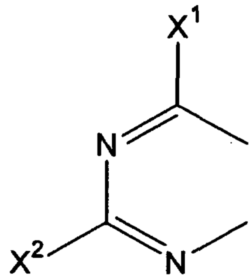

or

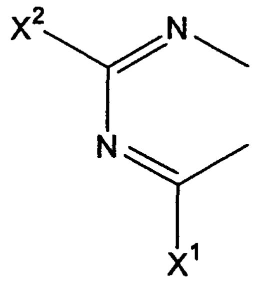

wherein $X^1$ and $X^2$ are each independently a halogen atom,

------ is a single bond or a double bond,

$R^1$ is an alkyl group optionally having substituent(s), an alkenyl group optionally having substituent(s), an alkynyl group optionally having substituent(s), an aryl group optionally having substituent(s) or a heteroaryl group optionally having substituent(s),

$R^2$ is absent, or a hydrogen atom, an alkyl group optionally having substituent(s), an alkenyl group optionally having substituent(s), an alkynyl group optionally having substituent(s), an aryl group optionally having substituent(s) or a heteroaryl group optionally having substituent(s), and

Z is an amino-protecting group, a sugar group or an alkyl group,

or a salt thereof.

[2] The compound of the above-mentioned [1], which is a compound represented by the formula (2):

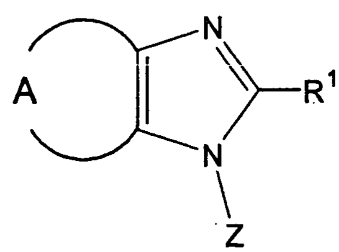 (2)

wherein

-A-, $R^1$ and Z are as defined in the above-mentioned [1], or a salt thereof.

[3] The compound of the above-mentioned [1], which is a compound represented by the formula (3):

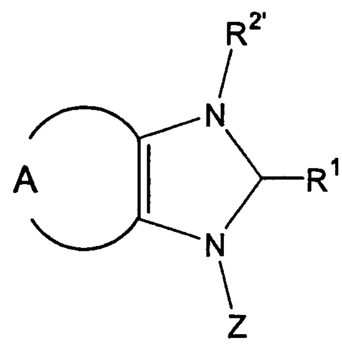

(3)

wherein

-A-, $R^1$ and Z are as defined in the above-mentioned [1], and
$R^{2'}$ is a hydrogen atom, an alkyl group optionally having substituent(s), an alkenyl group optionally having substituent(s), an alkynyl group optionally having substituent(s), an aryl group optionally having substituent (s) or a heteroaryl group optionally having substituent(s), or a salt thereof.

[4] The compound of the above-mentioned [3], which is a compound represented by the formula (4) :

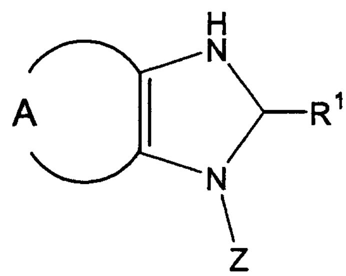

(4)

wherein

-A-, $R^1$ and Z are as defined in the above-mentioned [3], or a salt thereof.

[5] The compound of the above-mentioned [3], which is a compound represented by the formula (5):

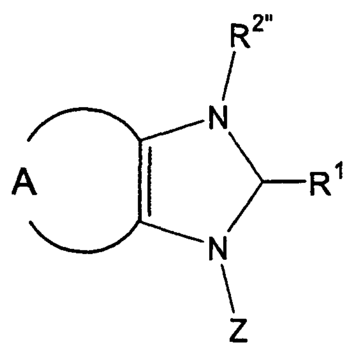

(5)

wherein

-A-, $R^1$ and Z are as defined in the above-mentioned [3], and
$R^{2''}$ is an alkyl group optionally having substituent(s), an alkenyl group optionally having substituent(s), an alkynyl group optionally having substituent(s), an aryl group optionally having substituent(s) or a heteroaryl group optionally having substituent(s), or a salt thereof.

[6] The compound of any one of the above-mentioned [1] to [5], wherein $X^1$ and $X^2$ are both chlorine atoms, or a salt thereof.
[7] The compound of any one of the above-mentioned [1] to [6], wherein Z is an amino-protecting group or a sugar group, or a salt thereof.
[8] The compound of the above-mentioned [7], wherein Z is benzyl, or a salt thereof.
[9] A production method of a compound of the above-mentioned [1] or a salt thereof, which comprises a step of reacting a compound represented by the formula (a):

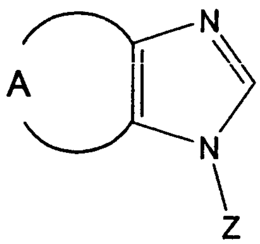

(a)

wherein

-A- is

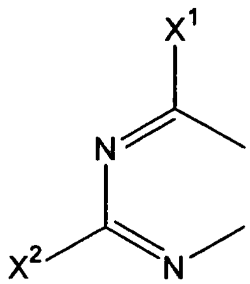

or

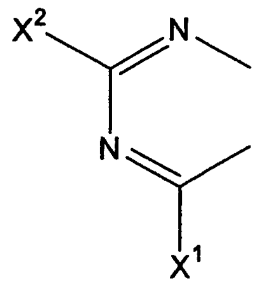

wherein $X^1$ and $X^2$ are each independently a halogen atom, and
Z is an amino-protecting group, a sugar group or an alkyl group, or a salt thereof, with an organometallic reagent.
[10] The method of the above-mentioned [9], wherein the organometallic reagent is a compound represented by the formula: $R^1Li$ wherein $R^1$ is an alkyl group optionally having substituent(s), an alkenyl group optionally having substituent(s), an alkynyl group optionally having substituent(s), an aryl group optionally having substituent(s) or a heteroaryl group optionally having substituent(s), or a compound represented by the formula: $R^1MgX$ wherein $R^1$ is an alkyl group optionally having substituent(s), an alkenyl group optionally having substituent(s), an alkynyl group optionally having substituent(s), an aryl group optionally having substituent(s) or a heteroaryl group optionally having substituent(s), and X is a chlorine atom, a bromine atom
or an iodine atom.
[11] The method of the above-mentioned [10], which further comprises an oxidation step using an oxidizing reagent.
[12] The method of the above-mentioned [11], wherein the oxidizing reagent is dichlorodicyano-*p*-benzoquinone, manganese dioxide or chloranil.
[13] The method of the above-mentioned [10], which further comprises a step of adding a compound represented by the formula: $R^{2''}L$ wherein $R^{2''}$ is an alkyl group optionally having substituent(s), an alkenyl group optionally having substituent(s), an alkynyl group optionally having substituent(s), an aryl group optionally having substituent (s) or a heteroaryl group optionally having substituent(s), and L is a leaving group.

[14] The method of any one of the above-mentioned [9] to [13], wherein Z is an amino-protecting group or a sugar group.

**Detailed Description of the Invention**

The present invention relates to a 2,6-dihalogeno-8-substituted-purine compound represented by the formula (I) :

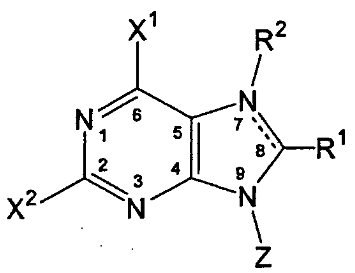
or
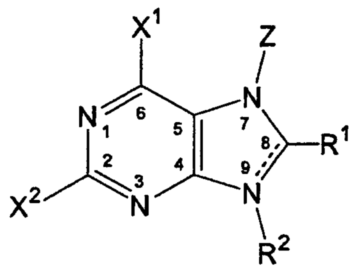
(I)

wherein

$R^1$, $R^2$ and Z are each as defined below,
$X^1$ and $X^2$ are each independently a halogen atom, and
------ is a single bond or a double bond (provided that when it is a double bond, $R^2$ is absent), or a salt thereof [hereinafter sometimes to be referred to as compound (I) in abbreviation], and a production method thereof. In the present specification, compound (I) is conveniently represented by the following formula (1) [hereinafter sometimes to be referred to as compound (1) in abbreviation].

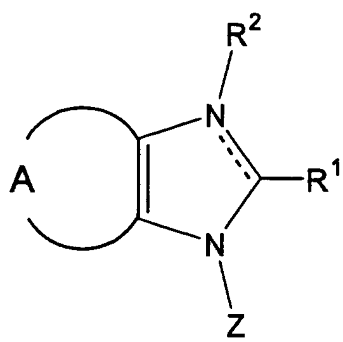
(1)

Therefore, -A- is

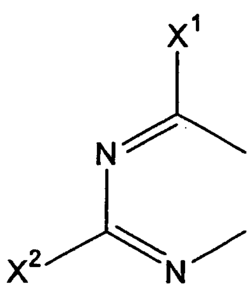
or
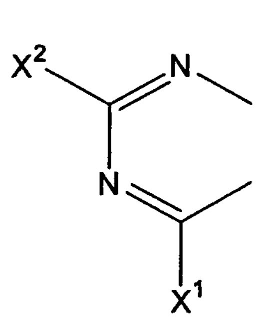

wherein $X^1$ and $X^2$ are each independently a halogen atom.

The above-mentioned compound (1) comprises compounds represented by the following formulae (2) and (3) [hereinafter sometimes to be referred to as compound (2) and (3) in abbreviation, respectively].

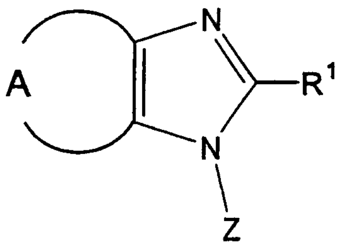
(2)

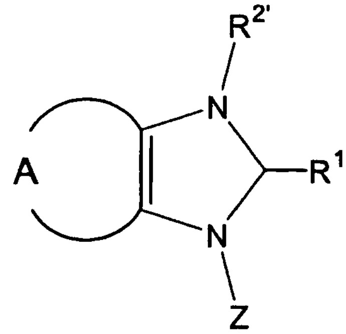
(3)

wherein -A- is as defined above, $R^1$, $R^{2'}$ and Z are each as defined below.

The above-mentioned compound (3) encompasses compounds represented by the following formulae (4) and the formula (5) [hereinafter sometimes to be referred to as compound (4) and (5) in abbreviation, respectively].

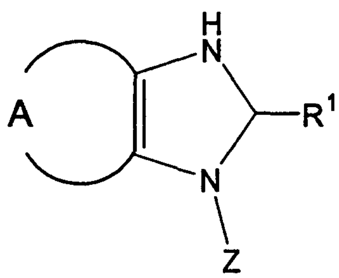
(4)

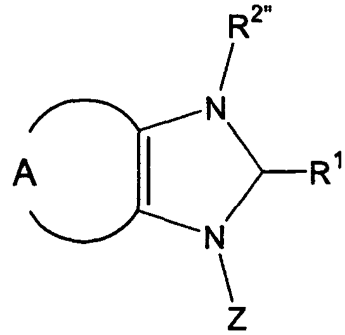
(5)

wherein -A- is as defined above, and $R^1$, $R^{2''}$ and Z are each as defined below.

The symbols and terms used in the present invention are defined in the following.

$R^1$ is an alkyl group optionally having substituent(s), an alkenyl group optionally having substituent(s), an alkynyl group optionally having substituent(s), an aryl group optionally having substituent(s) or a heteroaryl group optionally having substituent(s).

$R^2$ is absent, or a hydrogen atom, an alkyl group optionally having substituent(s), an alkenyl group optionally having substituent(s), an alkynyl group optionally having substituent(s), an aryl group optionally having substituent(s) or a heteroaryl group optionally having substituent(s).

$R^{2'}$ is a hydrogen atom, an alkyl group optionally having substituent(s), an alkenyl group optionally having substituent(s), an alkynyl group optionally having substituent(s), an aryl group optionally having substituent(s) or a heteroaryl group optionally having substituent(s).

$R^{2''}$ is an alkyl group optionally having substituent(s), an alkenyl group optionally having substituent(s), an alkynyl group optionally having substituent(s), an aryl group optionally having substituent(s) or a heteroaryl group optionally having substituent(s).

The "alkyl group" of the "alkyl group optionally having substituent(s)" for $R^1$, $R^2$, $R^{2'}$ or $R^{2''}$ is intended to mean a straight chain or branched chain or cyclic alkyl group having 1 to 10 carbon atoms.

As the straight chain or branched chain alkyl group, for example, alkyl having 1 to 10 carbon atoms (e.g., methyl, ethyl, *n*-propyl, isopropyl, *n*-butyl, isobutyl, sec-butyl, tert-butyl, pentyl, isopentyl, neopentyl, tert-pentyl, 1-methylbutyl, 2-methylbutyl, 1,2-dimethylpropyl, 1-ethylpropyl, hexyl, isohexyl, 1-methylpentyl, 2-methylpentyl, 3-methylpentyl, 1,1-dimethylbutyl, 1,2-dimethylbutyl, 2,2-dimethylbutyl, 1-ethylbutyl, 1,1,2-trimethylpropyl, 1,2,2-trimethylpropyl, 1-ethyl-2-methylpropyl, 1-ethyl-1-methylpropyl etc.) and the like can be mentioned.

As the cyclic alkyl group, for example, cycloalkyl having 3 to 10 carbon atoms (e.g., cyclopropyl, cyclobutyl,

cyclopentyl, cyclohexyl, cycloheptyl, cyclooctyl etc.) and the like can be mentioned.

The "alkenyl group" of the "alkenyl group optionally having substituent(s)" for $R^1$, $R^2$, $R^{2'}$ or $R^{2''}$ is intended to mean a straight chain or branched chain or cyclic alkenyl group having 2 to 10 carbon atoms.

As the straight chain or branched chain alkenyl group, for example, alkenyl having 2 to 10 carbon atoms (e.g., vinyl, allyl etc.) and the like can be mentioned.

As the cyclic alkenyl group, for example, cycloalkenyl having 5 to 10 carbon atoms (e.g., cyclopentenyl, cyclohexenyl etc.) and the like can be mentioned.

The "alkynyl group" of the "alkynyl group optionally having substituent(s)" for $R^1$, $R^2$, $R^{2'}$ or $R^{2''}$ is intended to mean a straight chain or branched chain alkynyl group having 2 to 10 carbon atoms, such as ethynyl and the like.

The "aryl group" of the "aryl group optionally having substituent (s) " for $R^1$, $R^2$, $R^{2'}$ or $R^{2''}$ is intended to mean an aryl group having 6 to 14 carbon atoms (e.g., phenyl, naphthyl, anthryl, biphenylyl etc.). Particularly, phenyl is preferable.

The "heteroaryl group" of the "heteroaryl group optionally having substituent(s)" for $R^1$, $R^2$, $R^{2'}$ or $R^{2''}$ is intended to mean a 5- to 8-membered heteroaryl group containing, as ring-constituting atom(s), 1 to 3 hetero atoms selected from the group consisting of a nitrogen atom, an oxygen atom and a sulfur atom besides carbon atoms (e.g., thienyl group, furyl group, pyranyl group, pyrrolyl group, pyridinyl group etc.).

As the "substituent" which the above-mentioned "alkyl group", "alkenyl group", "alkynyl group", "aryl group" and "heteroaryl group" each optionally have, a halogen atom (e.g., fluorine atom, chlorine atom, bromine atom, iodine atom), an alkyl group (e.g., a straight chain or branched chain or cyclic alkyl group having 1 to 10 carbon atoms defined in the above-mentioned "alkyl group", and the like), an alkoxy group (e.g., an alkoxy group having 1 to 10 carbon atoms [wherein the alkyl moiety of the alkoxy group is as defined for the straight chain or branched chain or cyclic alkyl group having 1 to 10 carbon atoms defined in the above-mentioned "alkyl group"] and the like), a cyano group, a nitro group, a carboxyl group, a silyl group having substituent(s) (e.g., the below-defined "silyl group having substituent(s)" such as trimethylsilyl group, dimethylphenylsilyl group and the like, and the like), an amino group, an alkylamino group (e.g., an alkylamino group having 1 to 10 carbon atoms [wherein the alkyl moiety of the alkylamino group is as defined for the straight chain or branched chain or cyclic alkyl group having 1 to 10 carbon atoms defined in the above-mentioned "alkyl group"] and the like), a perfluoroalkyl group (e.g., a perfluoroalkyl group having 1 to 10 carbon atoms such as trifluoromethyl group, pentafluoroethyl group and the like [wherein the alkyl moiety of the perfluoroalkyl group is as defined for the straight chain or branched chain or cyclic alkyl group having 1 to 10 carbon atoms defined in the above-mentioned "alkyl group"] and the like) and the like can be mentioned.

The kind and number of the substituents are not particularly limited and preferably have 1 to 5 substituents at the substitutable positions.

$X^1$ and $X^2$ are each independently a halogen atom.

The "halogen atom" for $X^1$ or $X^2$ is intended to mean a fluorine atom, a chlorine atom, a bromine atom or an iodine atom. Of these, a chlorine atom is preferable and more preferably, $X^1$ and $X^2$ are both chlorine atoms, from the aspect of the reactivity.

Z is an amino-protecting group, a sugar group or an alkyl group.

The "amino-protecting group" for Z is not particularly limited as long as it is an amino-protecting group known to those of ordinary skill in the art of the organic synthesis or well known. As the amino-protecting group to be used in the present invention, a heterocyclic group, an aralkyl group having 7 to 16 carbon atoms, an acyl group having 1 to 12 carbon atoms, a silyl group having substituent(s), an alkoxy-carbonyl group wherein the alkoxy has 1 to 6 carbon atoms, and the like can be preferably mentioned.

The "heterocyclic group" is intended to mean a group derived from 5- to 8-membered heterocycle containing, as ring-constituting atom(s), 1 to 3 hetero atoms selected from the group consisting of a nitrogen atom, an oxygen atom and a sulfur atom besides carbon atoms. For example, tetrahydropyranyl (e.g., tetrahydropyran-2-yl etc.), tetrahydrofuranyl (e.g., tetrahydrofuran-2-yl etc.) and the like can be mentioned. Of these, tetrahydropyranyl is preferable.

The "aralkyl group having 7 to 16 carbon atoms" is intended to mean an aralkyl group consisting of aryl having 6 to 10 carbon atoms (e.g., phenyl, naphthyl etc.) and alkyl having 1 to 6 carbon atoms (e.g., methyl, ethyl, *n*-propyl, isopropyl, *n*-butyl, isobutyl, sec-butyl, tert-butyl, pentyl, hexyl etc.), and, for example, benzyl and the like can be mentioned. Of these, benzyl is preferable.

The "acyl group having 1 to 12 carbon atoms" is intended to mean an acyl group such as formyl, alkylcarbonyl wherein the alkyl has 1 to 11 carbon atoms (e.g., acetyl, ethylcarbonyl, *n*-propylcarbonyl, isopropylcarbonyl, *n*-butylcarbonyl, isobutylcarbonyl, sec-butylcarbonyl, tert-butylcarbonyl etc.), arylcarbonyl wherein the aryl has 5 to 11 carbon atoms (e.g., benzoyl etc.) or heteroarylcarbonyl (e.g., 3-pyridylcarbonyl etc.) and the like.

The "silyl group having substituent(s)" is intended to mean a silyl group having any 3 substituents selected from the group consisting of alkyl having 1 to 6 carbon atoms and aryl having 6 to 10 carbon atoms, and the substituents on the silyl group may be the same or different. As the alkyl having 1 to 6 carbon atoms, which is a substituent for the "silyl group having substituent(s)", for example, methyl, ethyl, *n*-propyl, isopropyl, *n*-butyl, isobutyl, sec-butyl, tert-butyl,

pentyl, hexyl and the like can be mentioned. As the aryl having 6 to 10 carbon atoms, which is a substituent for the "silyl group having substituent(s)", phenyl and the like can be mentioned. As the "silyl group having substituent(s)", for example, trimethylsilyl, triisopropylsilyl, tert-butyldimethylsilyl, dimethylphenylsilyl, tert-butyldiphenylsilyl and the like can be mentioned.

The "alkoxy-carbonyl group wherein the alkoxy has 1 to 6 carbon atoms" is intended to mean an alkoxycarbonyl group consisting of alkoxy having 1 to 6 carbon atoms (e.g., methoxy, ethoxy, *n*-propoxy, isopropoxy, *n*-butoxy, isobutoxy, sec-butoxy, tert-butoxy, pentyloxy, hexyloxy etc.) and carbonyl, and, for example, *tert*-butoxy carbonyl and the like can be mentioned.

As the amino-protecting group for Z, a heterocyclic group and an aralkyl group having 7 to 16 carbon atoms are preferable, tetrahydropyranyl and benzyl are more preferable, and benzyl is most preferable, in view of the stability as a protecting group.

The "sugar group" for Z is not particularly limited as long as it is a sugar group known to those of ordinary skill in the art of the organic synthesis or well known. The sugar group to be used in the present invention is preferably a group derived from pentoses (including a group derived from furanoses, pyranoses and all isomers thereof, hydroxyl groups of the sugar are each independently optionally protected by a hydroxyl-protecting group, the carbon atom at 1-position of the sugar is directly attached to purine nucleus), particularly,

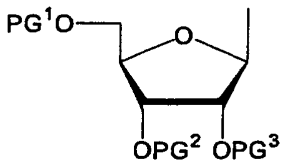 , 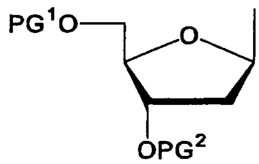 , 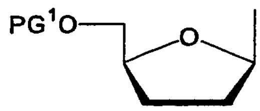 and 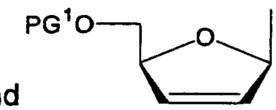

wherein $PG^1$, $PG^2$, $PG^3$ may be the same or different and each is independently a hydroxyl-protecting group or a hydrogen atom, are preferable.

The "hydroxy-protecting group" is not particularly limited as long as it is a hydroxy-protecting group known to those of ordinary skill in the art of the organic synthesis or well known. For example, an aralkyl group having 7 to 16 carbon atoms, an acyl group having 1 to 12 carbon atoms, a silyl group having substituent(s) and the like can be mentioned.

The "aralkyl group having 7 to 16 carbon atoms", "acyl group having 1 to 12 carbon atoms", "silyl group having substituent(s)" are each as defined for the "amino-protecting group" for Z above.

The "alkyl group" for Z is intended to mean a straight chain or branched chain or cyclic alkyl group having 1 to 10 carbon atoms defined for the "alkyl group" of the above-mentioned "alkyl group optionally having substituent(s)".

As Z, an amino-protecting group or a sugar group is preferable, an amino-protecting group is more preferable, and benzyl is particularly preferable.

L is a leaving group.

As the "leaving group" for L, for example, an iodine atom, a bromine atom, a chlorine atom, methanesulfonate group ($CH_3$-$SO_2$-O-), *p*-toluenesulfonate group (*p*-$CH_3$-$C_6H_4$-$SO_2$-O-), trifluoromethanesulfonate group ($CF_3$-$SO_2$-O-) and the like can be mentioned. Of these, an iodine atom is preferable from the aspects of the reactivity.

As the "organometallic reagent" to be used in the present invention, for example, organolithium reagent, Grignard reagent and the like, which are known to those of ordinary skill in the art or well known can be mentioned.

The "organolithium reagent" to be used in the present invention is a compound represented by the formula: $R^1Li$ wherein $R^1$ is as defined above. For example, phenyllithium, *n*-butyllithium, *tert*-butyllithium and the like can be mentioned.

The "Grignard reagent" to be used in the present invention is a compound represented by the formula: $R^1MgX$ wherein $R^1$ is as defined above and X is chlorine atom, bromine atom or iodine atom. For example, 4-chlorophenylmagnesium bromide and vinylmagnesium bromide and the like can be mentioned.

The "oxidizing reagent" to be used in the present invention is intended to mean an oxidizing agent, which is used for the organic synthesis and known to those of ordinary skill in the art or a well known (including oxygen). Of these, dichlorodicyano-*p*-benzoquinone (DDQ), manganese dioxide ($MnO_2$) and chloranil are preferable, and particularly, DDQ is more preferable from the aspects of the reactivity and solubility.

The "salt" of the compound of the present invention is not particularly limited and, for example, hydrochlorides, sulfates, nitrates, carbonates, methanesulfonates, *p*-toluenesulfonates, trifluoromethanesulfonates and the like can be mentioned.

A production method of compound (1) or a salt thereof is explained by referring to the scheme below. The following scheme aims at exemplarily show the production method according to the present invention, and does not

limit the production method according to the present invention to the methods shown in the scheme only.

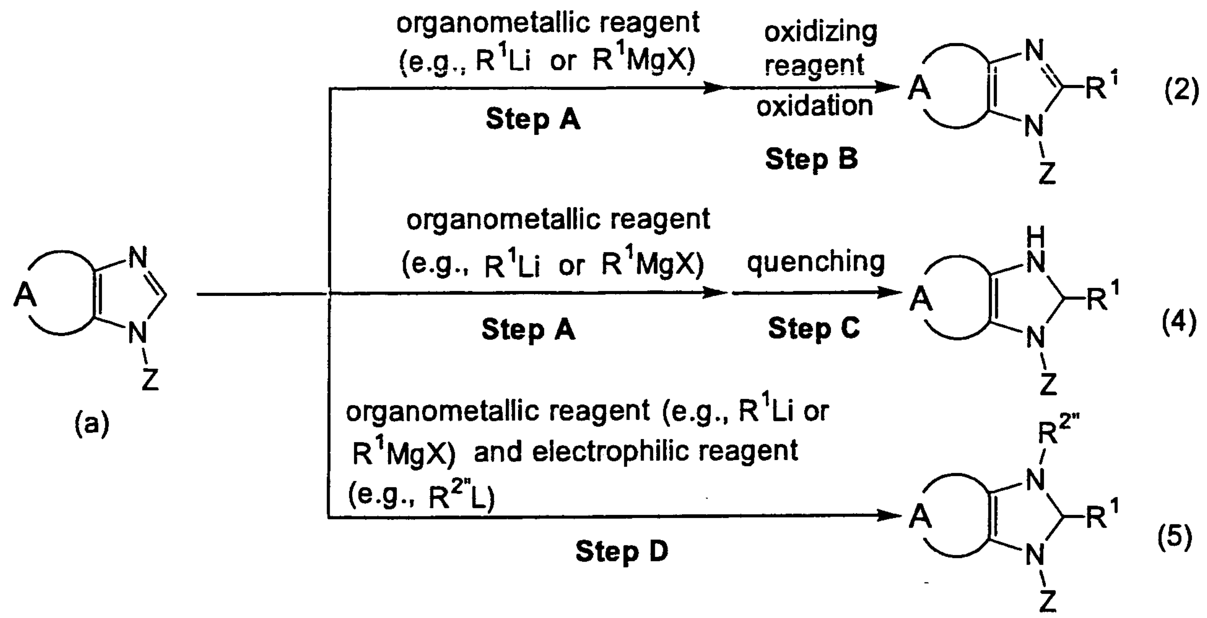


**Scheme**

In the scheme, each symbol is as defined above.

Each step in the scheme is explained in the following.

(Step A)

Step A is a step of reacting a 2,6-dihalogenopurine compound represented by the formula (a) wherein -A- and Z are as defined above, [hereinafter sometimes to be referred to as compound (a) in abbreviation] or a salt thereof with an organometallic reagent.

The compound (a) can be synthesized by a method known to those of ordinary skill in the art [e.g., a method described in the literature: G. Langli; L. L. Gundersen and F. Rise, Tetrahedron 1996, 52 (15), 5625-5638, and the like]. It can be easily derived from a commercially available purine compound.

As the salts of compound (a), for example, hydrochlorides, sulfates, nitrates, carbonates, methanesulfonates, *p*-toluenesulfonates, trifluoromethanesulfonates and the like can be mentioned.

As the organometallic reagent, organolithium reagent represented by the formula: $R^1Li$ wherein $R^1$ is as defined above, and Grignard reagent represented by the formula: $R^1MgX$, wherein $R^1$ and X are as defined above, can be preferably used.

The organometallic reagent can be prepared according to a method known to those of ordinary skill in the art. Alternatively, a commercially available product may be used.

The amount of the organometallic reagent to be used is 1.0 mol to 10 mol, preferably 1.0 mol to 3.0 mol, per 1 mol of compound (a) or a salt thereof.

The reaction solvent is not particularly limited as long as it does not adversely affect the reaction, and preferably includes aprotonic solvents, such as tetrahydrofuran (THF), diethyl ether, cyclohexane, methyl *tert*-butyl ether, toluene, dichloromethane, a mixed solvent thereof and the like.

The amount of the reaction solvent to be used is 100 mL to 100 L, preferably 1 L to 15 L, per 1 mol of compound (a).

In Step A, a solution containing an organometallic reagent [hereinafter sometimes to be referred to as adding solution] is desirably added (preferably added dropwise) to a solution in which compound (a) or a salt thereof has been dissolved in the above-mentioned reaction solvent [hereinafter sometimes to be referred to as subject solution], in view of easy handling.

The solvent, which can be used for the preparation of an adding solution, is not particularly limited as long as it does not adversely affect the reaction, and preferably includes aprotonic solvents, such as tetrahydrofuran (THF),

diethyl ether, cyclohexane, methyl *tert*-butyl ether, toluene, dichloromethane, a mixed solvent thereof and the like. It is desirable to use the same solvent as the reaction solvent.

The concentration and adding rate for the adding solution are not particularly limited as long as they do not adversely affect other reaction conditions.

While the temperature of the adding solution varies depending on the reaction conditions, it is generally -80°C to 50°C, preferably -80°C to 0°C.

While the temperature of the subject solution varies depending on the reaction conditions, it is generally -80°C to 50°C, preferably -80°C to 0°C.

While the reaction temperature varies depending on the reaction conditions, it is generally -80°C to 50°C, preferably -80°C to 0°C.

While the reaction time varies depending on the reaction conditions, it is generally 0.01 hr to 48 hrs, preferably 0.1 hr to 5 hrs.

The reaction of Step A is preferably carried out under conditions, for example, under nitrogen atmosphere, under argon atmosphere, and the like, for the purpose of avoiding decomposition of an organometallic reagent and improving the yield.

For example, when organolithium reagent represented by the formula: $R^1Li$ wherein $R^1$ is as defined above or Grignard reagent represented by the formula: $R^1MgX$ wherein $R^1$ and X are as defined above, is used as the organometallic reagent, it is considered that the following compound represented by the formula (b) wherein -A-, $R^1$, Z and X are respectively as defined above [hereinafter sometimes to be referred to as compound (b) in abbreviation] has been formed in the reaction system after the reaction.

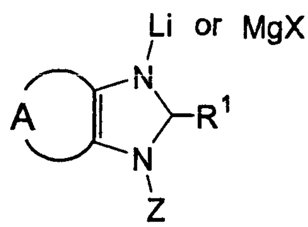

(b)

The substituent $R^1$ can be selectively introduced at the 8-position of compound (a).

After the completion of the reaction, the reaction mixture can be used as it is in an oxidization step (Step B explained below) or can be subjected to a quench step (Step C explained below).

(Step B)

Step B is an oxidation step using an oxidizing reagent.

As the oxidizing reagent, an oxidizing reagent known to those of ordinary skill in the art of the organic synthesis can be used, and specifically, dichlorodicyano-p-benzoquinone (DDQ), manganese dioxide ($MnO_2$) and chloranil are preferable, and DDQ is particularly preferable from the aspects of the solubility and reactivity.

The amount of the oxidizing reagent to be used is 0.1 mol to 10 mol, preferably 0.5 mol to 1.5 mol, per 1 mol of compound (a) or a salt thereof.

In Step B, a solution containing an oxidizing reagent [hereinafter sometimes to be referred to as oxidizing reagent solution] is desirably added (preferably added dropwise) to the reaction mixture obtained in Step A [hereinafter sometimes to be referred to as addition subject] from the aspects of easy handling.

The solvent usable for preparation of an oxidizing reagent solution is not particularly limited as long as it does not adversely affect the reaction, and preferably includes aprotonic solvents such as tetrahydrofuran (THF), diethyl ether, cyclohexane, methyl *tert*-butyl ether, dichloromethane, toluene, a mixed solvent thereof and the like.

The concentration and adding rate for the oxidizing reagent solution are not particularly limited as long as they do not adversely affect other reaction conditions.

While the temperature of the oxidizing reagent solution varies depending on the reaction conditions, it is generally -80 °C to 50 °C, preferably -80 °C to 0 °C.

While the temperature of the addition subject varies depending on the reaction conditions, it is generally -80 °C to 50 °C, preferably -80 °C to 0 °C.

While the reaction temperature varies depending on the reaction conditions, it is generally -80 °C to 50 °C, preferably -80 °C to 0 °C.

While the reaction time varies depending on the reaction conditions, it is generally 0.01 hr to 48 hrs, preferably 0.1 hr to 5 hrs.

After the completion of the reaction, compound (2) or a salt thereof can be obtained by a suitable work-up. The compound (2) or a salt thereof can be isolated and/or purified according to a conventional method, as necessary. In addition, the salt of compound (2) can be converted to other salts according to a method known to those of ordinary skill in the art.

(Step C)

Step C is a step of quenching the reaction of Step A.

The solution to be used for quenching is not particularly limited as long as it is a protonic solution (solvent containing $H^+$) capable of quenching the reaction of Step A. For example, water, saturated aqueous ammonium chloride solution, aqueous ammonium sulfate solution, aqueous acetic acid solution and the like can be mentioned. Of these, saturated aqueous ammonium chloride solution is preferable because it has a buffering effect and has a pH range allowing the object compound to be stable.

While the quenching temperature of the reaction varies depending on the reaction conditions, it is generally -80 °C to 100 °C, preferably -80 °C to 40 °C.

After quenching, compound (4) or a salt thereof can be obtained. The compound (4) or a salt thereof can be isolated and/or purified according to a conventional method, as necessary. In addition, the salt of compound (4) can be converted to other salts according to a method known to those of ordinary skill in the art.

The compound (4) or a salt thereof obtained in Step C may be subjected to the oxidation step of the above-mentioned Step B.

(Step D)

Step D is a step of reacting compound (a) or a salt thereof with an organometallic reagent and an electrophilic reagent.

The organometallic reagent used in Step D is as defined for the organometallic reagent used in the above-mentioned Step A. That is, as the organometallic reagent used in Step D, organolithium reagent represented by the formula: $R^1Li$ wherein $R^1$ is as defined above, and Grignard reagent represented by the formula: $R^1MgX$ [wherein $R^1$ and X are as defined above] are preferably used.

In Step D, as the electrophilic reagent, a compound represented by the formula: $R^{2''}L$ wherein $R^{2''}$ and L are respectively as defined above is used.

In Step D, compound (5) or a salt thereof can be obtained by reacting compound (a) or a salt thereof with an organometallic reagent and an electrophilic reagent.

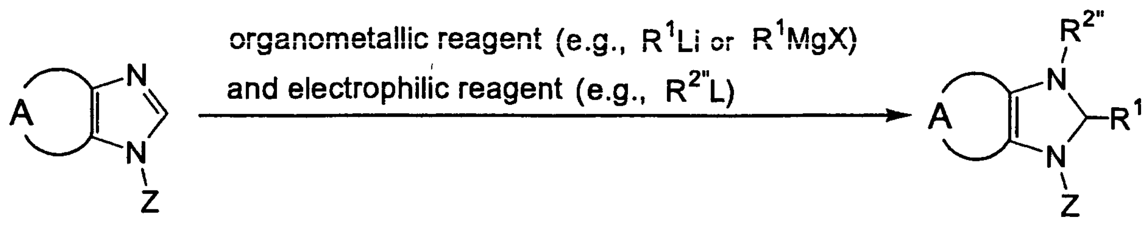


(a) (5)

In the reaction, when the following metal exchange reaction between an organometallic reagent and an electrophilic reagent proceeds:

$$R^1Li \text{ or } R^1MgX+R^{2''}L \rightarrow R^{2''}Li \text{ or } R^{2''}MgX+R^1L,$$

the following compound represented by the formula (6):

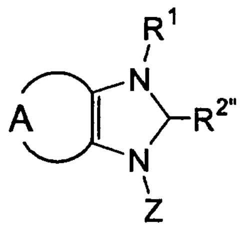

(6)

wherein each symbol is as defined above, or a salt thereof [hereinafter sometimes to be referred to as compound (6) in abbreviation] can be also obtained.

Alternatively, applying the above-mentioned metal exchange reaction, an organometallic reagent represented by the formula: $R^1Li$ or the formula: $R^1MgX$, and an electrophilic reagent represented by the formula: $R^{2''}L$ may be separately prepared. For example, the following metal exchange reaction is separately carried out under suitable conditions, and the resulting reaction mixture is reacted with compound (a) or a salt thereof to give compound (5) or a salt thereof.

$$R^{2''}Li \text{ or } R^{2''}MgX + R^1L \rightarrow R^1Li \text{ or } R^1MgX + R^{2''}L$$

In the scheme, the compound represented by the formula: $R^{2''}Li$ wherein $R^{2''}$ is as defined above, is an organometallic reagent (i.e., an organolithium reagent), and, for example, phenyllithium, *n*-butyllithium, tert-butyllithium and the like can be used.

In the scheme, the compound represented by the formula: $R^{2''}$ MgX wherein $R^{2''}$ is as defined above and X is a chlorine atom, a bromine atom or a iodine atom is an organometallic reagent (i.e., Grignard reagent) and, for example, 4-chlorophenylmagnesium bromide, vinylmagnesium bromide and the like can be used.

In the scheme, the compound represented by the formula: $R^1L$ [wherein $R^1$ and L are as defined above] is an electrophilic reagent.

In Step D, compound (5) or a salt thereof can be produced by (1) reacting compound (a) or a salt thereof with an organometallic reagent (e.g., $R^1Li$ or $R^1MgX$) in the same manner as in Step A above, and then (2) reacting the resultant with an electrophilic reagent (e.g., $R^{2''}L$).

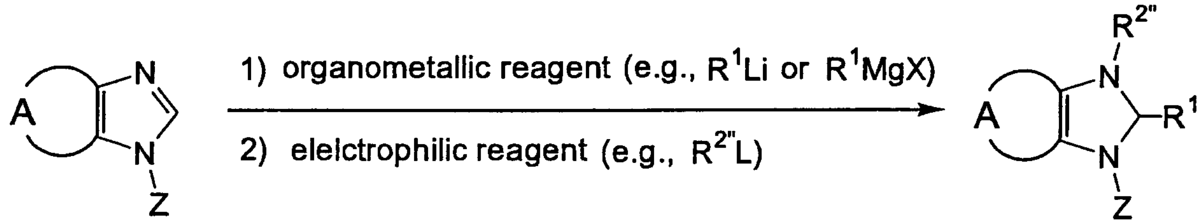


(a) (5)

The organometallic reagent and the electrophilic reagent used in Step D can be synthesized according to methods known to those of ordinary skill in the art. Alternatively, commercially available products may be used.

The amount of the organometallic reagent to be used is 1.0 mol to 10 mol, preferably 1.0 mol to 3.0 mol, per 1 mol of compound (a) or a salt thereof.

The amount of the electrophilic reagent to be used is 1.0 mol to 10 mol, preferably 1.0 mol to 3.0 mol, per 1 mol of compound (a) or a salt thereof.

The reaction solvent is not particularly limited as long as it does not adversely affect the reaction, and preferably includes aprotonic solvents such as tetrahydrofuran (THF), diethyl ether, cyclohexane, methyl *tert*-butyl ether, toluene, dichloromethane, a mixed solvent thereof and the like.

The amount of the reaction solvent to be used is 100 mL to 100 L, preferably 1 L to 15 L, per 1 mol of compound (a).

While the reaction temperature varies depending on the reaction conditions, it is generally -80 °C to 50 °C, preferably -80 °C to 0 °C.

While the reaction time varies depending on the reaction conditions, it is generally 0.01 hr to 48 hrs, preferably 0.1 hr to 5 hrs.

The reaction in Step D is preferably carried out under conditions, for example, under nitrogen atmosphere, under argon atmosphere and the like, for the purpose of avoiding decomposition of the organometallic reagent and improving the yield.

According to the above-mentioned Steps A-D, compound (2), (4), (5) and (6) or a salt thereof, namely, a 2,6-dihalogeno-8-substituted-purine compound represented by the formula (1) or a salt thereof can be easily produced from a 2,6-dihalogenopurine compound represented by the formula (a) or a salt thereof.

**Best Mode for Embodying the Invention**

The present invention is explained in detail in the following by referring to Reference Example and Examples. These Reference Example and Examples exemplarily show the present invention and do not limit the present invention in any way.

**Reference Example 1**

Synthesis of 9-benzyl-2,6-dichloro-9H-purine and 7-benzyl-2,6-dichloro-7H-purine

9-Benzyl-2,6-dichloro-9H-purine and 7-benzyl-2,6-dichloro-7H-purine were synthesized according to a method described in the literature: G. Langli; L. L. Gundersen and F. Rise, Tetrahedron 1996, 52 (15), 5625-5638.

2,6-Dichloropurine (18.9 g, 0.10 mol) and potassium carbonate (41.5 g, 0.30 mol) were added to DMF (500 mL) and the mixture was stirred under nitrogen atmosphere for 20 min. Benzylchloride (17.5 mL, 0.15 mol) was added and the mixture was further stirred for 24 hrs. After filtration, DMF was evaporated under reduced pressure, and the obtained reaction mixture was separated and purified by silica gel column chromatography to give 9-benzyl-2,6-dichloro-9H-purine (18.1 g, 64.8 mmol, yield 65%) and 7-benzyl-2,6-dichloro-7H-purine (2.79 g, 10.0 mmol, yield 10%).

**Example 1**

Synthesis of 9-benzyl-2,6-dichloro-8-phenyl-9H-purine

9-Benzyl-2,6-dichloro-9H-purine (84 mg, 0.30 mmol) was added to THF (4 mL) and the mixture was cooled to -78°C under argon. An organometallic reagent solution [phenyllithium (0.33 mmol), 1.9 M solution in cyclohexane - diethyl ether (7:3)] was added dropwise thereto, and the mixture was stirred for 5 min. After the completion of the reaction, 1M solution of DDQ (dichlorodicyano-*p*-benzoquinone, 0.2 mmol) in THF (2 mL) was added dropwise. After allowing to warm to room temperature, saturated aqueous $NH_4Cl$ solution (10 mL) was added, and the mixture was partitioned. The aqueous layer was extracted with $CH_2Cl_2$ (10 mL). The organic layers were combined, washed with saturated brine (10 mL) and dried over $MgSO_4$. The organic solvent was evaporated under reduced pressure, and the obtained residue was separated and purified by silica gel column chromatography to give the title compound (55 mg, 0.16 mmol, 53%).
$^1$H-NMR (250MHz, $CDCl_3$) : 5.45 (s,2H), 6.91-6.98 (m,2H), 7.16-7.23 (m,3H), 7.35-7.51 (m,3H), 7.54-7.60 (m,2H).
$^{13}$C-NMR (62.5MHz, $CDCl_3$): 47.7, 126.7, 128.1, 128.3, 128.9, 129.0, 129.3, 130.4, 131.3, 134.9, 150.7, 152.4, 155.0, 157.1.
MS(EI,70eV) 354($M^+$,12%), 292 (33%), 134(74%), 91(100%).

**Example 2**

Synthesis of 9-benzyl-8-(4'-chlorophenyl)-2,6-dichloro-9H-purine

The title compound (9.4 mg, 0.024 mmol, 8%) was obtained by a method similar to that of Example 1 and using 4-chlorophenylmagnesium bromide (0.33 mmol) as an organometallic reagent.
$^1$H-NMR (500MHz, $CDCl_3$): 5.45 (s,2H), 6.95-6.97 (m,2H), 7.20-7.25 (m,3H), 7.39 (d,2H,J=8.5Hz), 7.53 (d,2H, J=8.5Hz). $^{13}$C-NMR (125MHz, $CDCl_3$): 48.2, 127.1, 128.9, 129.6, 129.8, 130.9, 131.1, 135.3, 138.3, 139.2, 151.5, 153.2, 155.6, 156.4.
MS(EI,70eV) 388($M^+$,4%), 134(52%), 91(100%).

**Example 3**

Synthesis of 9-benzyl-8-*n*-butyl-2,6-dichloro-9H-purine

The title compound (10.0 mg, 0.033 mmol, 10%) was obtained by a method similar to that of Example 1 and using *n*-butyllithium (0.33 mmol) as an organometallic reagent.
$^1$H-NMR (250MHz, $CDCl_3$): 0.76-0.85 (m,3H), 1.24-1.42 (m,2H), 1.57-1.72 (m,2H), 2.84-2.96 (t,2H), 5.75 (s,2H), 7.04-7.40 (m,5H).
$^{13}$C-NMR (125MHz, $CDCl_3$): 12.6, 21.4, 27.0, 28.0, 45.5, 126.0, 127.6, 128.2, 128.9, 133.6, 148.7, 151.0, 153.7, 158.8.
MS(EI,70eV) 291(22%), 277(8%), 91(100%).

Example 4

Synthesis of 7-benzyl-2,6-dichloro-8,9-dihydro-8-vinyl-7H-purine

7-Benzyl-2,6-dichloro-7H-purine (84 mg, 0.30 mmol) was added to THF (4 mL) and the mixture was cool to -78°C under argon. A solution of vinylmagnesium bromide, as an organometallic reagent [0.33 mmol, 1.0 M solution in THF] was added dropwise thereto, and the mixture was stirred for 5 min. After allowing to warm to room temperature, saturated aqueous $NH_4Cl$ solution (10 mL) was added, and the mixture was partitioned. The aqueous layer was extracted with $CH_2Cl_2$ (10 mL). The organic layers were combined, washed with saturated brine (10mL) and dried over $MgSO_4$. The organic solvent was evaporated under reduced pressure, and the obtained residue was separated and purified by silica gel column chromatography to give the title compound (51 mg, 0.16 mmol, 55%).
$^1$H-NMR (500MHz, $CDCl_3$): 4.13 (d,1H,J=16.1Hz), 5.02 (d,1H,J=16.1Hz), 5.28 (d,1H,J=17.2Hz), 5.32 (d,1H,J=9.8Hz), 5.53 (d,1H,J=8.2Hz), 5.76 (dt,1H,J=9.0,17.2Hz), 7.23 (t,1H,J=7.4Hz), 7.28 (t,1H,J=7.4Hz), 8.16 (d,2H,J=7.4Hz), 8.37 (br,1H).
$^{13}$C-NMR (125MHz, $CDCl_3$): 48.0, 80.1, 122.1, 126.0, 127.2, 128.3, 128.4, 129.3, 134.3, 136.8, 146.3, 162.3.

**Industrial Applicability**

According to the present invention, a 2,6-dihalogeno-8-substituted-purine compound or a salt thereof, which is useful as an intermediate for producing medicaments, can be produced easily from a 2,6-dihalogenopurine compound or a salt thereof. Therefore, a 2,6-dihalogeno-8-substituted-purine compound or a salt thereof can be easily provided.
This application is based on a patent application No. 2003-016667 filed in Japan, the contents of which are hereby all incorporated by reference.

**Claims**

1. A compound represented by the formula (1) :

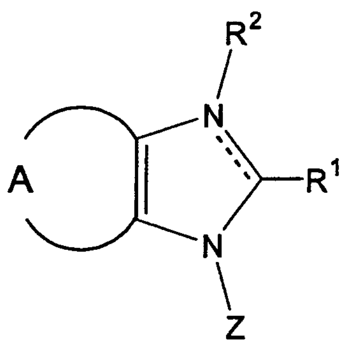

(1)

wherein

-A- is

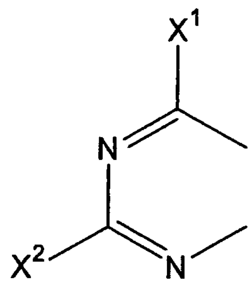

or

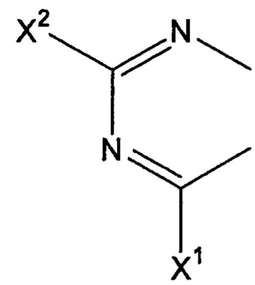

wherein $X^1$ and $X^2$ are each independently a halogen atom,

------ is a single bond or a double bond,
$R^1$ is an alkyl group optionally having substituent(s), an alkenyl group optionally having substituent(s), an alkynyl group optionally having substituent(s), an aryl group optionally having substituent(s) or a heteroaryl group optionally having substituent(s),
$R^2$ is absent, or a hydrogen atom, an alkyl group optionally having substituent(s), an alkenyl group optionally having substituent(s), an alkynyl group optionally having substituent(s), an aryl group optionally having substituent(s) or a heteroaryl group optionally having substituent(s), and
Z is an amino-protecting group, a sugar group or an alkyl group,

or a salt thereof.

**2.** The compound of claim 1, which is a compound represented by the formula (2):

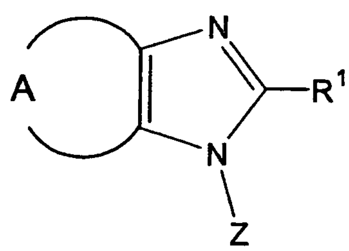

(2)

wherein

-A-, $R^1$ and Z are as defined in claim 1, or a salt thereof.

**3.** The compound of claim 1, which is a compound represented by the formula (3):

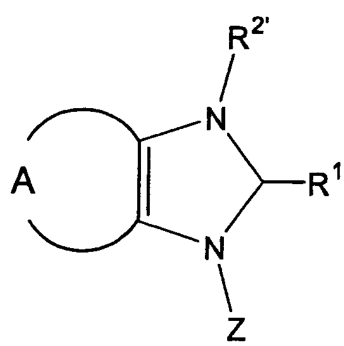

(3)

wherein

-A-, $R^1$ and Z are as defined in claim 1, and
$R^{2'}$ is a hydrogen atom, an alkyl group optionally having substituent(s), an alkenyl group optionally having substituent(s), an alkynyl group optionally having substituent(s), an aryl group optionally having substituent (s) or a heteroaryl group optionally having substituent(s), or a salt thereof.

**4.** The compound of claim 3, which is a compound represented by the formula (4):

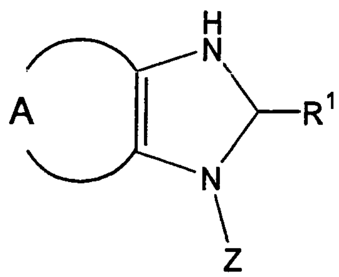

(4)

wherein

-A-, $R^1$ and Z are as defined in claim 3, or a salt thereof.

**5.** The compound of claim 3, which is a compound represented by the formula (5):

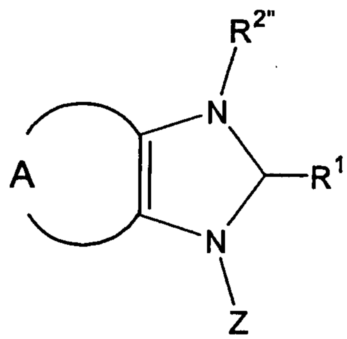

(5)

wherein

-A-, $R^1$ and Z are as defined in claim 3, and
$R^{2''}$ is an alkyl group optionally having substituent(s), an alkenyl group optionally having substituent(s), an alkynyl group optionally having substituent(s), an aryl group optionally having substituent(s) or a heteroaryl group optionally having substituent(s), or a salt thereof.

**6.** The compound of any one of claims 1 to 5, wherein $X^1$ and $X^2$ are both chlorine atoms, or a salt thereof.

**7.** The compound of any one of claims 1 to 6, wherein Z is an amino-protecting group or a sugar group, or a salt thereof.

**8.** The compound of claim 7, wherein Z is benzyl, or a salt thereof.

**9.** A production method of a compound of claim 1 or a salt thereof, which comprises a step of reacting a compound represented by the formula (a):

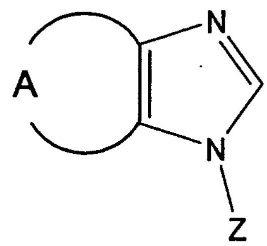

(a)

wherein

-A- is

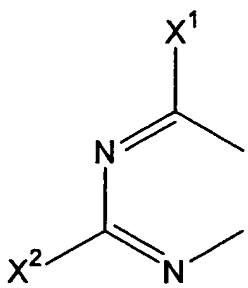

or

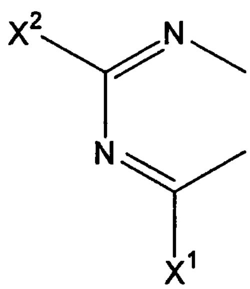

wherein $X^1$ and $X^2$ are each independently a halogen atom, and
Z is an amino-protecting group, a sugar group or an alkyl group, or a salt thereof, with an organometallic reagent.

**10.** The method of claim 9, wherein the organometallic reagent is a compound represented by the formula: $R^1Li$ wherein $R^1$ is an alkyl group optionally having substituent(s), an alkenyl group optionally having substituent(s), an alkynyl group optionally having substituent(s), an aryl group optionally having substituent(s) or a heteroaryl group optionally having substituent(s), or a compound represented by the formula: $R^1MgX$ wherein $R^1$ is an alkyl group optionally having substituent(s), an alkenyl group optionally having substituent(s), an alkynyl group optionally having substituent(s), an aryl group optionally having substituent(s) or a heteroaryl group optionally having substituent(s), and X is a chlorine atom, a bromine atom or an iodine atom.

**11.** The method of claim 10, which further comprises an oxidation step using an oxidizing reagent.

**12.** The method of claim 11, wherein the oxidizing reagent is dichlorodicyano-*p*-benzoquinone, manganese dioxide or chloranil.

**13.** The method of claim 10, which further comprises a step of adding a compound represented by the formula: $R^{2''}L$ wherein $R^{2''}$ is an alkyl group optionally having substituent(s), an alkenyl group optionally having substituent(s), an alkynyl group optionally having substituent(s), an aryl group optionally having substituent(s) or a heteroaryl group optionally having substituent(s), and L is a leaving group.

**14.** The method of any one of claims 9 to 13, wherein Z is an amino-protecting group or a sugar group.

## INTERNATIONAL SEARCH REPORT

International application No.
PCT/JP03/14703

**A. CLASSIFICATION OF SUBJECT MATTER**
Int.Cl[7] C07D473/40

According to International Patent Classification (IPC) or to both national classification and IPC

**B. FIELDS SEARCHED**

Minimum documentation searched (classification system followed by classification symbols)
Int.Cl[7] C07D473/40

Documentation searched other than minimum documentation to the extent that such documents are included in the fields searched

Electronic data base consulted during the international search (name of data base and, where practicable, search terms used)
REGISTRY(STN), CAPLUS(STN), CAOLD(STN), CASREACT(STN)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | EP 1251130 A1 (KYOWA HAKKO KOGYO CO., LTD), 23 October, 2002 (23.10.02), Reference example 36<br>& WO 01/47931 A1 & AU 2001022235 A<br>& US 2003/176698 A1 | 1,2,6-8<br>3,4,9-12,14<br>5,13 |
| X<br>Y<br>A | GUTOROV, L.A., et al., Syntheses in the purine series., XX. Effect of chloride compounds of phosphorus on 8-methyltheobromine, Khimiko-Farmatsevticheskii Zhurnal, 1969, Vol.3, No.7, pages 4 to 10; compounds II, VII | 1,2,6<br>3,4,9-12<br>5,13 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 February, 2004 (03.02.04) | 24 February, 2004 (24.02.04) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

International application No.
PCT/JP03/14703

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Hitoshi HARADA et al., 2-Alkynyl-8-aryl-9-methyladenines as Novel Adenosine Receptor Antagonists: Their Synthesis and Structure-Activity Relationships toward Hepatic Glucose Production Induced via Agonism of the $A_{2B}$ Receptor, J.Med.Chem., 2001, Vol.44, No.2, pages 170 to 179; compounds 13a-n | 1,2<br>5 |
| X<br>A | US 2002/0058671 A1 (PFIZER INC.),<br>16 May, 2002 (16.05.02),<br>Example 1<br>& US 2002/061897 A1 & US 2003/100546 A1 | 1,2,6,7<br>5 |
| Y<br>A | Thomas C. McKENZIE, et al., The Reaction of 6-Halopurines with Phenyl Metal Complexes, J. Heterocyclic.Chem., 1987, Vol.24, pages 1551 to 1553; full text | 3,4,9-12,14<br>13 |
| Y<br>A | Eisaku HAYASHI et al., "Purine Yudotai ni Kansuru Kenkyu (Dai 1 Po), 9-Phenyl-9H-purine Oyobi 7-Phenyl-7-Hpurine to Grignard Shiyaku tono Hanno ni Tsuite", Journal of the Pharmaceutical Society of Japan, 1979, Vol.99, No.2, pages 114 to 119; full text | 3,4,9-12,14<br>13 |
| Y<br>A | WO 93/17021 A1 (PFIZER INC.),<br>02 September, 1993 (02.09.93),<br>Preparations A, C<br>& JP 7-500115 A & EP 626964 A1<br>& FI 943798 A & US 5583137 A | 3,4,9-12,14<br>5,13 |

Form PCT/ISA/210 (continuation of second sheet) (July 1998)